(19) **Europäisches Patentamt**
**European Patent Office**
**Office européen des brevets**

(11) **EP 0 629 849 B1**

(12) **EUROPÄISCHE PATENTSCHRIFT**

(45) Veröffentlichungstag und Bekanntmachung des Hinweises auf die Patenterteilung:
**09.09.1998   Patentblatt 1998/37**

(51) Int Cl.6: **G01N 7/00**, B01D 5/00, A61L 2/24

(21) Anmeldenummer: **94108974.0**

(22) Anmeldetag: **10.06.1994**

(54) **Verfahren zur Messung nichtkondensierbarer Gase in Sterilisierdampf**

Method for measuring of non-condensible gases in sterilisation vapours

Procédé de mesure des gaz non-condensables dans la vapeur de stérilisation

(84) Benannte Vertragsstaaten:
**AT BE CH DE ES FR IT LI PT**

(30) Priorität: **14.06.1993  DE 4319402**

(43) Veröffentlichungstag der Anmeldung:
**21.12.1994   Patentblatt 1994/51**

(73) Patentinhaber: **C. STIEFENHOFER GmbH**
**D-86971 Peiting (DE)**

(72) Erfinder: **Kammermeier, Bernhard, Dipl.-Ing.**
**D-82396 Fischer/Ammersee (DE)**

(74) Vertreter: **Schaumburg, Thoenes, Thurn**
**Patentanwälte**
**Postfach 86 07 48**
**81634 München (DE)**

(56) Entgegenhaltungen:
**DE-A- 2 846 826          DE-A- 3 636 716**
**GB-A- 586 530**

## Beschreibung

Die Erfindung betrifft ein Verfahren und eine Vorrichtung zur Messung des Anteils von nicht kondensierbaren Gasen nach dem Obergriff des Anspruchs 1.

Ein solches Verfahren ist aus DE-A-3636716 bekannt.

Alle dort beschriebenen Ausführungsbeispiele sind schmutzempfindlich und dadurch nicht besonders zuverlässig. Insbesondere das System mit der Lichtschranke ist sehr empfindlich gegenüber Ablagerungen an der Meßrohrinnenwand. Bei allen Ausführungsbeispielen ist eine feinmechanische Präzision in der Fertigung erforderlich. Eine direkte Auswertung des Meßsignals ist nicht möglich. Für Integralbildung über Durchschaltzeiten der Lichtschranke ist z. B. ein Rechner erforderlich. Alle vorgestellten Ausführungsbeispiele sind schwer zu justieren und schwer zu kontrollieren.

Ferner ist aus der DE-A-2846826 ein Verfahren der im Oberbegriff des Anspruchs 1 beschriebenen Art bekannt, bei dem die Verminderung des Volumens einer Flüssigkeitssäule proportional zur Länge der im Steigrohr eingeschlossenen und die Flüssigkeit verdrängenden Luftblasen als Meßgröße benutzt wird.

Der Erfindung liegt die Aufgabe zugrunde, die beschriebenen Nachteile zu vermeiden und eine Vorrichtung der genannten Art

1. absolut schmutzunempfindlich auszubilden
2. den Einsatz von Standardbauteilen zu ermöglichen
3. eine direkte Auswertung des Meßsignals zu ermöglichen
4. eine sehr einfache Justierung und Kontrolle des Meßsignals zu ermöglichen.

Diese Aufgabe wird erfindungsmäßig gelöst mit einer Vorrichtung nach dem kennzeichnenden Teil des Anspruchs 1 und sowie den Ausgestaltungen weiterer Ansprüche.

Vorteile der Erfindung sind die absolute Schmutzunempfindlichkeit, die Möglichkeit des Einsatzes von Standardbauteilen, die Verwendung des Gewichts von Flüssigkeitssäulen als Meßgröße, die durch Druckmessung eine direkte Auswertung des Meßsignals ermöglicht. Die Justierung und Kontrolle des Meßsignals ist auf einfache Weise möglich, da die Längen der Luftblasen im Steigrohr mit einem Längenmeßgerät nachgemessen werden können.

Ausführungsbeispiele der Erfindung sind in den Abbildungen dargestellt und werden im folgenden näher beschrieben.

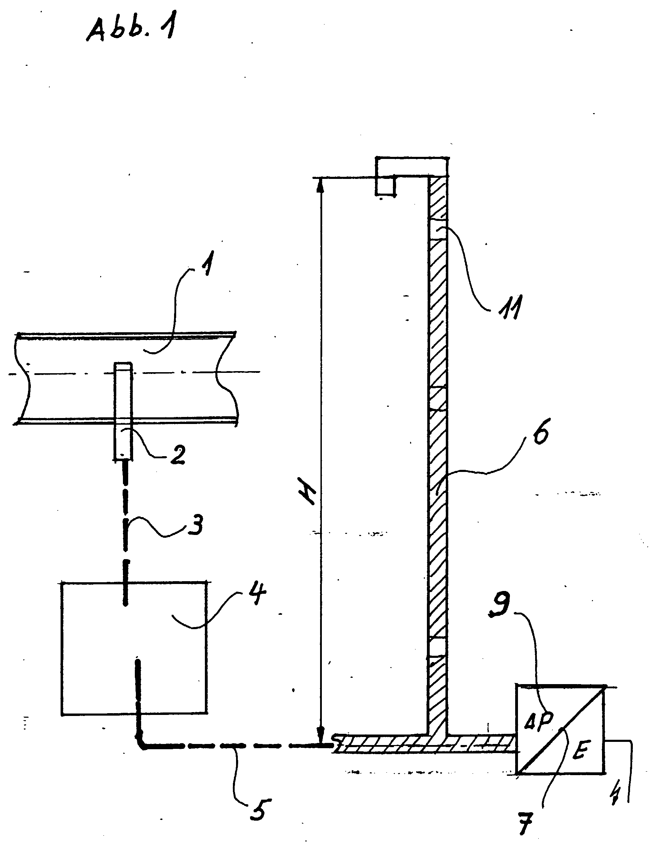

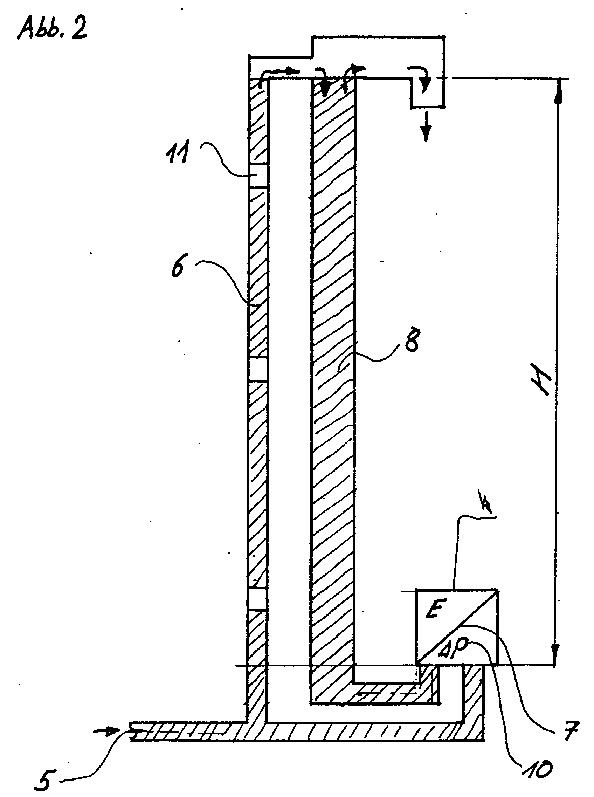

Es zeigen Abbildung 1 eine erfindungsmäßige Vorrichtung mit einem senkrechten Meßrohr, Abbildung 2 eine erfindungsmäßige Vorrichtung mit einem senkrechten Meßrohr und einem zusätzlichen senkrechten Vergleichsrohr.

Aus einer Dampfleitung 1 wird mittels einer Entnahmevorrichtung 2 während des Verfahrensablaufs kontinuierlich oder absatzweise ein vorzugsweise geringer Volumenstrom Dampfgasgemisches entnommen. Das Gas-Dampf-Gemisch wird über eine Leitung 3 zu einem Kühler 4 geführt. Vom Kühler 4 wird das aus dem Kühler austretende Kondensat-Gas-Gemisch über Leitung 5 dem Meßsystem zugeführt. Das Meßsystem besteht aus einer vorzugsweise senkrecht angeordneten Meßleitung 6 mit einem oberen Überlauf und einem unten an die Meßleitung angeschlossenen Druckmeßgerät 7. Bei der Anordnung nach Abbildung 1 ist das Meßsignal die Differenz zwischen dem sich aus der geodätischen Höhe ergebenden Druck einer Kondensatsäule ohne Gaseinschluß und dem wirklich gemessenen Druck 9. Das Meßsystem wird so ausgeführt, daß die Fließgeschwindigkeit im Steigrohr niedrig ist (ca. 0,1 m/ s), um die Meßfehler durch Rohrreibungsverluste gering zu halten.

Die Meßleitung 6 ist im Querschnitt so eng gewählt, daß der Stokes'sche Auftrieb nur schwach wirksam wird und die Blasen 11 aus nicht kondensierbaren Gasen deshalb nur so langsam aufsteigen können, daß die Gleichmäßigkeit des Meßsignals nicht verfälscht werden kann.

In Abbildung 2 ist parallel zur weitgehend senkrechten Meßleitung 6 eine ebenfalls weitgehend senkrecht angeordnete Vergleichsleitung 8 dargestellt. Die Vergleichsleitung 8 ist mit einem weiten Querschnitt ausgebildet, so daß der Stokes'sche Auftrieb voll wirksam werden kann und somit Gasblasen, sofern solche dort hingelangen sollten, schnell aufsteigen können und deshalb die Kondensatsäule in der Vergleichsleitung praktisch gasfrei ist. Der Überlauf der Meßleitung 6 mündet oben in die Vergleichsleitung 8. Dadurch wird erreicht, daß aus der Meßleitung 6 ausfließendes Kondensat zuerst in die Vergleichsleitung 8 fließt, diese füllt und immer gefüllt hält und erst dann, wenn die Vergleichsleitung voll ist, durch den Überlauf ins Freie abfließen kann. Das Meßsignal bildet hier die direkt ablesbare Druckdifferenz 10 zwischen dem Druck der Vergleichsleitung 8 und dem durch den Einfluß von Gasblasen 11 in der Meßleitung 6 auftretenden geringeren Druck.

Der Meßwert wird in Volumenprozent, bezogen auf den kondensierten Zustand des Dampf-Gas-Gemisches, angegeben.

$$\text{Meßwert \%} = \frac{\text{Volumen der nichtkondensierbaren Gase}}{\text{Gesamtvolumen von Gas und Kondensat}} \times 100$$

Der Meßwert bezieht sich auf Atmosphärendruck (Normdruck) und eine definierte Temperatur, z. B. 70° C.

Da das Steigrohr über die ganze Länge einen einheitlichen Innendurchmesser aufweist, entspricht die Gewichtsverringerung der Wassersäule und damit die gemessene Druckverringerung direkt der Volumenverdrängung durch die gasgefüllten Rohrabschnitte. Der zunächst als Drucksignal erfaßte Meßwert kann somit ohne Umrechnung als Volumen-Prozent-Anteil angegeben werden.

Falls eine besonders hohe Meßgenauigkeit erforderlich ist, muß der schwankende Umgebungsdruck berücksichtig werden, da dieser Einfluß auf die Volumenverdrängung durch die Gasblasen hat. Dazu muß der Umgebungsdruck mit einem separaten Absolutdrucksensor gemessen werden und eine rechnerische Korrektur des Meßsignals über die allgemeinen Gasgesetze vorgenommen werden.

Geringfügige Meßfehler entstehen auch dadurch, daß Luftblasen, die sich noch im unteren Bereich des Meßrohres befinden, durch die darüber befindliche Säule geringfügig komprimiert und damit die Volumenverdrängung verringert wird. Dieser Meßfehler kann bei den meisten Fällen vernachlässigt werden, aber auch rechnerisch teilweise kompensiert werden, indem bei der beschriebenen Kompensation des Umgebungsdrucks auch die halbe Steigrohrhöhe (Mittelwert) berücksichtigt wird.

Da auch die Temperatur Einfluß auf die Volumenverdrängung durch die Gasblasen und auch auf das Gewicht der Wassersäule hat, kann ein Temperatursensor im Steigrohr eingesetzt werden und das Meßsignal über die Gasgesetze auf eine bestimmte Bezugstemperatur umgerechnet werden.

## Patentansprüche

1. Verfahren zur Messung des Anteils von nicht kondensierbaren Gasen und Dämpfen, insbesondere in Sterilisierdampf eines Sterilisators, wobei während des Verfahrensablaufes aus dem Dampfsystem ein Teil des Dampf-Gas-Gemisches abgezogen, in einem Kühler kondensiert und in ein Meßsystem geleitet wird, **dadurch gekennzeichnet**, daß die Differenz des Druckes einer aus Kondensat und nicht kondensierbaren Gasen bestehenden und in einem Steigrohr (6) eingeschlossenen Fluidsäule gegenüber dem Druck einer gleich hohen Kondensatsäule ohne Gas gemessen wird.

2. Verfahren nach Anspruch 1, **dadurch gekennzeichnet**, daß die Kondensationsleitung und die als Steigrohr (6) ausgebildete Meßleitung so eng im Querschnitt gewählt sind, daß der Stockes'sche Auftrieb nur schwach wirksam wird und die Blasen aus nicht kondensierbaren Gasen deshalb nur so langsam aufsteigen können, daß die Gleichmäßigkeit des Meßsignals nicht verfälscht werden kann.

3. Verfahren nach Anspruch 2, **dadurch gekennzeichnet**, daß parallel zur als Steigrohr (6) ausgebildeten Meßleitung mit engem Querschnitt eine ebenfalls als Steigrohr ausgebildete Vergleichsleitung (8) mit weitem Querschnitt angeordnet ist und daß die Druckdifferenz zwischen dem unteren Ende der Meßleitung (6) und den unteren Ende der Vergleichsleitung (8) mittels eines angeschlossenen Differenzdruckmessers gemessen wird.

4. Verfahren nach Anspruch 3, **dadurch gekennzeichnet**, daß die Meßleitung (6) am oberen Ende der Vergleichsleitung (8) in diese mündet, so daß das aus der Meßleitung (6) ausfließende Kondensat zuerst in die Vergleichsleitung (8) fließt, diese auffüllt und ständig gefüllt hält und anschließend durch einen Überlauf ins Freie abfließt.

5. Verfahren nach einen der Ansprüche 1 bis 4, **dadurch gekennzeichnet**, daß eine Temperaturkompensation über eine Temperaturmessung vorgesehen ist.

6. Verfahren nach einem der Ansprüche 1 bis 5, **dadurch gekennzeichnet**, daß eine Kompensation des Umgebungsdruckes vorgesehen ist.

## Claims

1. A method for measuring the portion of non-condensible gases and vapours, in particular in the sterilisation vapour of a sterilizing apparatus, wherein during the course of the process a part of the vapour-gas-mixture is drawn off from the vapour system, condensed in a condenser and conducted into a measuring system, **characterized** in that the difference in pressure between a fluid column consisting of condensate and non-condensible gases and being occluded in an ascending pipe (6) and the pressure of a condensate column without gas having the same height is measured.

2. A method according to claim 1, **characterized** in that the condensation conduit and the measuring conduit formed as ascending pipe (6) are chosen as narrow in cross-section that the Stokes' buoyancy takes effect only weakly and therefore the bubbles of the non-condensible gases can only ascend so slow that the uniformity of the measuring signal can not be distorted.

3. A method according to claim 2, **characterized** in that a comparison conduit (8) also formed as ascending pipe and having a wide cross-section is arranged in parallel to the measuring conduit formed as ascending pipe (6) and having a narrow cross-section and that the difference in pressure between the lower end of the measuring conduit (6) and the lower end of the comparison conduit (8) is measured by means of a connected differential pressure gauge.

4. A method according to claim 3, **characterized** in that the measuring conduit (6) leads into the comparison conduit (8) at the upper end thereof so that the condensate flowing out of the measuring conduit (6) first flows into the comparison conduit (8), fills it up and keeps it constantly filled and afterwards flows into the open via an overflow.

5. A method according to any one of the claims 1 to 4, **characterized** in that a temperature compensation via a temperature measurement is provided.

6. A method according to any one of the claims 1 to 5, **characterized** in that a compensation of the ambient pressure is provided.

**Revendications**

1. Procédé de mesure de la fraction de vapeurs et de gaz non condensables, en particulier dans la vapeur de stérilisation d'un stérilisateur, selon lequel, lors de la mise en oeuvre du procédé, une partie du mélange gaz - vapeurs est prélevée du système de vapeur, condensée dans un condenseur et dirigée dans un système de mesure, caractérisé par la mesure de la différence entre la pression d'une colonne de fluide, enfermée dans un conduit ascendant (6) et constituée de condensat et de gaz non condensables, et la pression d'une colonne de condensat de même hauteur sans gaz.

2. Procédé selon la revendication 1, caractérisé en ce qu'il est choisi un conduit de condensation et un conduit de mesure formant conduit ascendant (6) qui sont de section étroite, de telle sorte que la poussée de Stokes ne devient que faiblement active et que les bulles de gaz non condensables ne peuvent par conséquent que monter lentement, de sorte que l'uniformité du signal de mesure ne peut pas être altérée.

3. Procédé selon la revendication 2, caractérisé en ce qu'un conduit de comparaison (8), de section large, formant également conduit ascendant, est agencé parallèlement au conduit de mesure formant conduit ascendant (6) , et caractérisé par la mesure de la différence de pression entre l'extrémité inférieure du conduit de mesure (6) et l'extrémité inférieure du conduit de comparaison (8) au moyen d'un manomètre différentiel auquel les extrémités sont raccordées.

4. Procédé selon la revendication 3, caractérisé en ce que le conduit de mesure (6) débouche dans le conduit de comparaison (8) à l'extrémité supérieure de celui-ci, de sorte que le condensat sortant du conduit de mesure (6) s'écoule tout d'abord dans le conduit de comparaison (8), puis le remplit continûment jusqu'à s'écouler à l'air libre par un organe de débordement.

5. Procédé selon l'une des revendications 1 à 4, caractérisé en ce qu'il est prévu une compensation en température de la mesure de la température.

6. Procédé selon l'une des revendications 1 à 5, caractérisé en ce qu'il est prévu une compensation de la pression ambiante.

Abb. 1

Abb. 2